# EUROPÄISCHE PATENTSCHRIFT

(11) **EP 0 504 781 B1**
(45) Veröffentlichungstag und Bekanntmachung des Hinweises auf die Patenterteilung: **29.01.1997**
(21) Anmeldenummer: 92104532.4
(22) Anmeldetag: 17.03.1992
(51) Int. Cl.: A61F 2/02

(54) **Implantierbares biohybrides Organ**
Bio-hybrid implantable organ
Organe bio-hybride implantable

(30) Priorität: 18.03.1991 DE 4108772
(43) Veröffentlichungstag der Anmeldung: 23.09.1992
(73) Patentinhaber: DEUTSCHE INSTITUTE FÜR TEXTIL- UND FASERFORSCHUNG STUTTGART Stiftung des öffentlichen Rechts, 73770 Denkendorf (DE)
(72) Erfinder: Planck, Heinrich, Dipl.-Ing. Dr., W-7440 Nürtingen (DE); Dauner, Martin, Dipl.-Ing., W-7300 Esslingen (DE); Doser, Michael, Dipl.-Bio. Dr., W-7024 Filderstadt (DE); Renardy, Monika, Dipl.-Biol., W-7000 Stuttgart (DE); Zschocke, Peter, Dipl.-Chem. Dr., W-7406 Mössingen (DE)
(74) Vertreter: Patentanwälte Ruff, Beier, Schöndorf und Mütschele

(56) Entgegenhaltungen:
- WO-A-82/03764
- WO-A-91/02498
- DE-A- 3 422 639
- US-A- 4 309 776
- US-A- 5 024 671
- LIFE SUPPORT SYSTEMS. Bd. 1, Nr. 3, Juli 1983, EASTBOURNE GB Seiten 189 - 196; J.B. BUCHS ET AL.: 'BIOTECHNICAL ASPECTS OF A NEW HYBRID..'

## Beschreibung

Gegenstand der Erfindung ist ein biohybrides Hohlorgan nach dem Oberbegriff des Anspruchs 1. Ein solches Hohlorgan ist aus der DE-A-3422639 bekannt. Auch die WO91/02498 und der Text "Biotechnical Aspects of a New Hybrid Artificial Endocrine Pancreas" im Life Support Systems, Vol. 1 (1983), beschreiben Hohlorgane, die zwar von dem aus der DE-A-3422639 strukturell abweichen, jedoch vom Prinzip der ähnlich sind.

Organe, z.B. Niere, Leber, Pankreas und andere Drüsen des Körpers sind nicht durch rein synthetische Prothesen implantierbar zu ersetzen. Der bislang einzige Weg zum Ersatz von Organen ist die Transplantation kompletter Organe oder u. U. funktioneller Einheiten des Organs. Die Transplantation ist jedoch aufgrund der geringen Verfügbarkeit von geeigneten Spendern, des Problems der immunologischen Abstoßung des transplantierten Organs und aufgrund operationstechnischer Probleme nur in viel zu geringem Umfang durchführbar.

Ein Ausweg ist die Verwendung biohybrider Organe, bestehend aus einem polymeren Träger, meist einer Membran, verbunden mit lebenden Zellen. Die Zellen können autolog, homolog oder heterolog sein. Die polymere Membran erfüllt unterschiedliche Aufgaben. Sie fungiert zum einen als rein mechanischer Träger. Zusätzlich oder gleichzeitig kann die Membran eine immunologische Schutzfunktion ausüben, um die verwendeten Zellen vor der Immunabwehr des Empfängers zu schützen.

Die infolge der Häufigkeit der Erkrankungen und der Problematik der derzeit durchgeführten Behandlungsmöglichkeiten wichtigsten Organe für einen biohybriden Organersatz sind insbesondere Niere, Pankreas und Leber. Die Funktion der Niere wird heute durch rein künstliche Systeme (künstliche Niere) ersetzt. Für den Ersatz von Leber und Pankreas sind biohybride Organe in der Entwicklung.

### Stand der Technik

### 1. Extrakorporale Systeme

Zur kurzfristigen Behandlung von akutem Leberversagen werden derzeit extrakorporale Bioreaktor-Systeme entwickelt, bei denen isolierte Hepatocyten oder Stückchen von Lebergewebe in einem Membranreaktor die Entgiftung des durch diesen Reaktor geleiteten Blutes durchführen [(Matsumura K.N. et al: Hybrid artificial liver in hepatic failure. Preliminary clinical report, Surgery 101, 1 (1987), 99 - 103)]. Diese extrakorporalen Bioreaktor-Systeme haben einige gravierende Nachteile:
1) Die Patienten müssen stationär an den Reaktor angeschlossen werden, sind also in ihrer Bewegungsfreiheit stark eingeschränkt.
2) Die im Membranreaktor eingeschlossenen Hepatocyten oder Leberfragmente sind nur über eine begrenzte Zeit funktionsfähig und müssen dann ausgetauscht werden.

Für einen längerfristigen Einsatz sind diese Systeme daher nicht sinnvoll.

### 2. Implantierbare Systeme

Bei der Entwicklung eines implantierbaren biohybriden Pankreas oder einer implantierbaren biohybriden Leber werden 2 unterschiedliche Systeme angewandt, die Mikroverkapselung und die Makroverkapselung.

### Mikroverkapselung

Bei der Mikroverkapselung werden einzelne Zellen, z.B. Hepatocyten, im Fall des biohybriden Pankreas einzelne Langerhans'sche Inseln, in Membranen eingeschlossen und intraperitoneal transplantiert [(Sun A.M. et al: Microencapsulated hepatocytes as a bioartificial liver, Trans. Am. Soc. Artif. Int. Organs 32 (1986), 39 - 41; Lim F. et al: Microencapsulated islets as bioartificial endocrine pancreas, Science 210 (1980), 908 - 912; Iwata H. et al: Evaluation of encapsulated Langerhans islets in polyion complex microbeads as a bioartificial pancreas, Transactions 3rd World Biomaterials Congress Kyoto (1988), 291; Sefton M.V. et al: Submerged extrusion for the microencapsulation of mammalian cells in poly-HEMA-MMA, Transactions 3rd World Biomaterials Congress Kyoto (1988), 294; Dupuy B. et al: In situ polymerisation of a microencapsulating medium round living cells, J. Biomed. Mater. Res. 22 (1988), 1061 - 1070)]. Der Erfolg der Transplantation mikroverkapselter Inseln bei der Behandlung diabetischer Versuchstiere ist sehr unterschiedlich. Ein großes Problem ist die auftretende, häufig sehr starke Fibrose rund um die Mikrokapseln [(Darquy S. et al: Comparative study of microencapsulated rat islets implanted in different diabetic models in mice, Methods in Islet Transplantation Research, Thieme (1990), 209 - 214)], durch die der im Peritoneum grundsätzlich durch lange Diffusionsstrecken nicht optimale Stoffaustausch zwischen Inseln und Blut weiter behindert wird. Ein weiteres Problem ist eine auftretende immunologische Abwehrreaktion, die durch aus einem Teil der Mikrokapseln durch Leakage freigesetzten Inseln hervorgerufen wird [(Bretzel, R.G.: Workshop on "Immunoisolation of Islets of Langerhans", 1988)]. Dieselben Probleme treten bei der Transplantation mikroverkapselter Hepatocyten auf.

### Makroverkapselung

Für die Makroverkapselung werden im allgemeinen marktgängige Membranen aus dem Bereich Dialyse, Plasmaphorese und Oxigenierung verwendet, die den Anforderungen bzgl. der molekularen Ausschlußgrenze genügen, aber in keiner Hinsicht an die Anwendung in einem biohybriden Pankreas speziell angepaßt sind.

### a) Extravaskuläre Systeme

Beim extravaskulären System werden die Zellen bzw. Langerhans'schen Inseln zwischen zwei am Umfang zusammengeklebte oder verschweißte Flachmembranen eingebracht. Zwischen den beiden Membranen kann auch ein ringförmiger Abstandhalter angeordnet sein, auf dessen Flachseiten die Membranen fixiert sind [(Strautz R.L.: Studies of hereditary-obese mice after implantation of pancreatic islets in millipore filter capsules, Diabetol. 6 (1970), 306 - 312; Zekorn T. et al: Islet transplantation in experimental diabetes of the rat. XI. in vitro tests on artificial membranes applied in islet transplantation, Horm. Met. Res. 19 (1987), 87 - 88)]. Die Zellen werden über eine radiale Bohrung im Ring mittels einer Spritze eingebracht.

Außerdem können auch Membranhohlfasern als extravaskuläre Systeme verwendet werden.

Die extravaskulären Systeme werden als biohybrides Pankreas intraperitoneal [(Altman et al: Implantation of semi-permeable hollow fibers to prevent immune rejection of transplanted pancreatic islets, Horm. Met. Res. Suppl. 12 (1982), 43 - 45; Klomp, G.F. et al: Hydrogels for encapsulation of pancreatic islet cells, Trans. Am. Soc. Artif. Inter. Organs 25 (1979), 74 - 76; Valente U. et al: Human pancreatic islet transplantation in diffusion chambers, Horm. Met. Res. Suppl. 12 (1982), 48 - 51)], subcutan [(Valente U. et al: Human pancreatic islet transplantation in diffusion chambers, Horm. Met. Res. Suppl. 12 (1982), 48 - 51)], in Leber [(Zekorn T. et al: Bioartificial pancreas: The use of different hollow fibers as a diffusion chamber, Transplantation Proceedings 21 (1989), 2748 - 2750; Altman et al: Implantation of semi-permeable hollow fibers to prevent immune rejection of transplanted pancreatic islets, Horm. Met. Res. Suppl. 12 (1982), 43 - 45)], Milz [(Altman et al: Implantation of semi-permeable hollow fibers to prevent immune rejection of transplanted pancreatic islets, Horm. Met. Res. Suppl. 12 (1982), 43 - 45)] und Omentum [(Araki Y. et al: Normalization of blood glucose in totally pancreatectomized dogs by use of pancreatic chambers, Endocrinol. Japan. 27, 2 (1980), 157 - 161)] implantiert.

Der Stoffaustausch erfolgt durch Diffusion ins umliegende Gewebe und dort vorhandene Blutkapillaren. Infolge der geringen Vaskularisierung der Implantate entstehen sehr lange Diffusionsstrecken, was eine verzögerte Regulation des Blutzuckerspiegels zur Folge hat und in der anderen Richtung zu einer mangelnden Versorgung der enkapsulierten Inseln mit Nährstoffen und Sauerstoff führt. Die häufig um die Implantate auftretende Fibrose verschärft diese Probleme weiter, so daß ein längerfristiger Einsatz dieser Systeme zur Blutzuckerregulation nicht möglich ist.

### b) Systeme mit Anschluß an den Blutkreislauf

Diese Systeme bestehen aus einer oder mehrerer Membranhohlfasern, auf deren Außenseite die Hepatocyten oder Langerhans'schen Inseln lokalisiert sind, innen durch die Membranhohlfaser fließt das Blut [(Wolf C.F.W.: Cells cultured on artificial capillaries and their use as a liver assist device, Artif. Organs 4,4 (1980), 279 - 284; Tze W.J. et al: Implantable artificial capillary unit for pancreatic islet allograft and xenograft, Diabetol. 16 (1979), 247 - 252; Chick W.L. et al: Beta cell culture on synthetic capillaries: An artificial endocrine pancreas, Science 187 (1975), 847 - 849; Sun A.M. et al: The use, in diabetic rats and monkeys, of artificial capillary units containing cultured islets of Langerhans (artificial endocrine pancreas), Diabetes 26 (1977), 1136 - 1139; Tze W.J. et al: Studies with implantable artificial capillary units containing rat islets on diabetic dogs, Diabetol. 19 (1980), 541 -545; Naber S.P. et al: Progress in development of a hybrid artificial endocrine pancreas, Toxl. Med. Int. Congr. Series 580 (1980), 227 - 231; Reach G. et al: Functional evaluation of a bioartificial pancreas using isolated islets perifused with blood ultrafiltrate, Diabetes 30 (1981), 296 - 301; Reach G. et al: A u-shaped bioartificial pancreas with rapid glucose-insulin kinetics. In vitro evaluation and kinetic modelling, Diabetes 33 (1984), 752 - 761)].

Ungelöst ist bei allen beschriebenen Systemen das Problem der Thrombogenität der Membranoberfläche. Neben der Thrombogenität erfolgen an den Membranflächen weitere Interaktionen zwischen zirkulierendem Blut und der Membran, wie z.B. Komplementaktivierung, so daß Systeme in dieser Form für einen Einsatz beim Menschen nicht in Frage kommen.

### Erfindung

Es wurde gefunden, daß die wesentlichen Probleme der biohybriden Hohlorgane, die auch als bioartifizielle Organe bezeichnet werden, dadurch vermieden werden können, indem das Hohlorgan so ausgebildet wird, daß es in die Wandung oder als Wandung einer Leitungsbahn des Empfängers oder einer künstlich zu schaffenden Leitungsbahn eingesetzt wird. Dabei werden unter Leitungsbahnen vorzugsweise solche verstanden, die mindestens zeitweise mit Flüssigkeit durchströmt sind. Hierzu gehören insbesondere Blutgefäße. Es kommen aber auch andere Leitungsbahnen wie Lymphgefäße, Darmabschnitte in Frage. Auch die Speiseröhre, die mindestens zeitweise mit Flüssigkeiten durchströmt ist, kann als Einsatzort in Frage kommen. Bevorzugt sind Blutgefäße, insbesondere deshalb, weil die meisten Organe für die teilweise oder gänzlich ein Ersatz geschaffen werden soll, normalerweise blutdurchströmt sind.

Gegenstand der Erfindung ist somit ein biohybrides Hohlorgan gemäß Anspruch 1.

Durch ein solches Hohlorgan kann ein intensiver Stoffaustausch zwischen dem flüssigen Medium, in dem sich die Organzellen befinden, und der Flüssigkeit, die durch die Leitungsbahn strömt, stattfinden. Der Diffusionsweg ist außerordentlich gering. Durch die in der Regel starke Strömung in der Leitungsbahn wird ein starkes Konzentrationsgefälle erhalten, das die Diffusiongeschwindigkeit bei der ohnehin kurzen Diffusionsstrecke erhöht. Hinzu kommt, daß das Hohlorgan, da es auf die Wandung der Leitungsbahn aufgelegt oder anstelle eines Wandabschnittes oder eines Längsabschnittes der Leitungsbahn eingesetzt wird, den Querschnitt der Leitungsbahn praktisch nicht verengt und dadurch die Durchströmung der Leitungsbahn nicht beeinträchtigt, wenn dies nicht aus anderen Gründen beabsichtigt ist, z.B. zur Erzeugung einer Turbulenz. Das erfindungsgemäße Hohlorgan kann so ausgebildet sein, daß die mindestens eine Kammer bzw. eine Mehrzahl von Kammern, die ggf. miteinander in Verbindung stehen können, die geometrische Form des Hohlorgans bilden. In diesem Falle sind die Kammern ausreichend steif ausgebildet, daß das daraus gebildete Hohlorgan als Wandungsausschnitt bzw. - abschnitt, z.B. als Fenster oder Patch oder als ganzer Längsabschnitt der Leitungsbahn verwendbar ist. Bei einer anderen Ausführungsform ist es jedoch bevorzugt, einen Träger, insbesondere einen textilen Träger, vorzusehen, der die geometrische Struktur für den Einsatz in der Wandung oder als Wandung aufweist, wobei auf der dem Inneren der Leitungsbahn zuweisenden Innenseite des Trägers die Membranen der Kammern bzw. die Kammern selbst angeordnet sind.

Der textile Träger kann aus Faservlies z.B. gemäß Patent DE 28 06 030 oder Patent DE 25 34 935 C2 oder einer anderen textilen Struktur, insbesondere Gewirk, Gestrick, Gewebe oder Geflecht bestehen. Es kann aus einem oder mehreren körperverträglichen Polymerwerkstoffen bestehen, z.B. aus den Gruppen der Polyurethane, thermoplastischen Polyester, Polyolefine oder Polyfluorethylene, oder aus resorbierbaren Polymeren, die dann durch körpereigenes Bindegewebe ersetzt werden.

Die die Membranen aufweisenden Kammern bzw. als Hohlkörper ausgebildeten Membransysteme können als vorzugsweise flache Kammern oder Leitungen ausgebildet sein, deren in die als Einsatzort vorgesehenen Leitungsbahn weisende Seite von der Membran gebildet wird.

Vorzugsweise sind die Kammern oder Leitungen vollständig von Membranwandungen umgeben bzw. aus solchen gebildet.

Die Membranen bestehen in der Regel aus einem oder mehreren Polymerwerkstoffen, z.B. aus den Gruppen Polyacryletherketone, Polysulfone, Polycarbonate oder Celluloseacetate. Solche Membranen bzw. Membransysteme sind beispielsweise bei der Ultrafiltration bekannt.

Die Membranen können die Form von Flachmembranen und insbesondere von leitungsförmigen Membranen, wie rohr- bzw. schlauchförmigen Membranen und insbesondere von Kapillarmembranen haben, wobei die Rohr-, Schlauch- und Kapillarmembranen einen kreisförmigen, ovalen, trilobalen, triangel- oder trapezförmigen Querschnitt besitzen. Der Membranquerschnitt von leitungsförmigen Membranen liegt zwischen 0,001 mm² bis 15 mm², insbesondere bei ca. 1mm². Die Wandstärke liegt vorzugsweise bei 1 µm bis 300 µm, insbesondere 10 bis 200 µm. Der lichte Abstand zwischen Kammerwandungen ist etwas größer als die Größe der aufnehmenden Organzellen. Er beträgt in der Regel das 1,1-fache bis maximal 20-fache der Zellgröße, vorzugsweise das 1,1-fache bis 5-fache. Normalerweise ist eine möglichst große Nähe der Zellwandungen zu den Kammerwandungen erwünscht, so daß das 1,5-fache bis 2-fache an lichtem Abstand nicht überschritten wird.

Das oder die Membransysteme sind vorzugsweise innenseitig auf dem textilen Träger angeordnet, wobei folgende Anordnungen bevorzugt sind, insbesondere in Form einer einschichtigen Weise.
- Axiale Anordnung einer Vielzahl einzelner Kapillarmembransysteme, mit oder ohne Zwischenraum.
- Wendelförmige oder helixartige, insbesondere doppelhelixartige, Anordnung eines oder mehrerer Kapillarmembransysteme, mit oder ohne Zwischenraum.
- Vorzugsweise wendelförmig oder helixartige, insbesondere doppelhelixartige, Anordnung eines oder mehrerer Kapillarmembransysteme in einer Plissierung einer Gefäßprothese. Bei der wendelförmigen Anordnung ist ein paralleler Verlauf der einzelnen Membransystem bevorzugt.

Die Membranen sind speziell für den einzelnen Anwendungsfall optimiert im Bezug auf ihre Trenneigenschaften, d.h. Durchlässigkeit für die physiologisch essentiellen Substanzen bei gleichzeitiger Undurchlässigkeit für Immunglobuline. Z.B. ist für ein biohybrides Pankreas ein molekularer Cut-Off der Membran zwischen 50 und 120 kD sinnvoll, sowie im Bezug auf ihre Diffusionseigenschaften, z.B. beim biohybriden Pankreas auf einen raschen Durchgang von Glucose und Insulin. Der Cut-Off liegt vorzugsweise allgemein unter 140 kD.

Der Durchmesser von Kapillarmembranen ist vorzugsweise der Größe der Zellen bzw. Zellinseln angepaßt, die in die Kapillare eingefüllt werden. Optimal ist ein geringfügig über der Zellgröße bzw. Zellinselgröße liegender Kapillarinnendurchmesser, z.B. beim biohybriden Pankreas 500 - 600 µm.

Bei einer besonders bevorzugten Ausführungsform ist zur Vermeidung der an der Grenzfläche Prothese/Blut auftretenden Probleme, wie z.B. Komplementaktivierung und Thrombogenität, die innenseitige, dem Blutstrom zugewandte, Membranoberfläche mit vorzugsweise ortsständigen Zellen, wie Epithelzellen oder Endothelzellen besiedelt. Die Diffusion durch die Membranen wird dadurch nicht nennenswert beeinträchtigt. Es können körpereigene oder auch andere, z.B. embryonale Endothelzellen verwendet werden. Die Endothelzellen können direkt oder mittels Haftvermittler (funktionelle Gruppen, Haftungsfaktoren, wie z.B. Fibronectin) auf die Prothesenoberfläche aufgebracht sein. Die Zellbesiedlung kann auch durch die zusätzliche Besiedlung mit anderen Zellen, z.B. Fibroblasten, verbessert werden. Die Zellbesiedlung kann unmittelbar vor der Implantation erfolgen oder es kann zwischen Zellbesiedlung und Implantation eine in vitro-Kultivierung erfolgen, z.B. bis zur Erreichung eines stabilen, die Membran-Oberfläche vollständig bedeckenden Zellrasens. Die mit Zellen besiedelten Hohlorgane können in Kulturmedium, insbesondere in Serum, gelagert oder auch in eingefrorenem Zustand aufbewahrt werden.

Die Membransysteme können mit den für den jeweiligen Einsatzort funktionellen Zellen, z.B. Langerhans'schen Inseln für ein biohybrides Pankreas oder Hepatocyten für eine künstliche Leber, befüllt werden. Es kann auch ein kombiniertes biohybrides Organ vorgesehen sein, bei dem mehrere Membransysteme aus einem oder mehreren Polymerwerkstoffen mit verschiedenen funktionellen Zellen, z.B. Langerhans'schen Inseln und Hepatocyten, befüllt werden, wobei die verschiedenen Membransysteme für die unterschiedlichen Zelltypen bzgl. Cut-Off und Durchmesser des Membransystems an die Anforderungen der jeweiligen Zellen angepaßt sind. Es ist z.B. eine flächenmäßige, insbesondere längenmäßige Unterteilung des Hohlorgans entsprechend der erforderlichen Zellzahl der verschiedenen funktionellen Zellen möglich.

Die Kammern bzw. Membransysteme weisen Zuleitungen bzw. Ableitungen zum Befüllen und Entleeren auf. Solche Zuführungen zu den Membransystemen können direkt an der Außenseite des Hohlorganes enden, oder sie können entsprechend lang ausgebildet sein und in einen leicht zugänglichen Hohlraum des Körpers, z.B. in die Bauchhöhle, oder nach außen durch die Haut geführt werden. Der Verschluß der Zuführungen ist reversibel, um einen Austausch der im Membransystem eingeschlossenen Zellmasse zu ermöglichen.

Das Hohlorgan wird vorzugsweise als Gefäßprothese, A-V-Shunt oder an eine andere Stelle des Körpers mit hohem Flüssigkeits-, insbesondere Blutdurchsatz implantiert. Das Membransystem kann zusätzlich noch pharmazeutische Wirksubstanzen enthalten.

### Vorteile der Erfindung gegenüber bereits bekannten Systemen

1) Die verwendeten Membranen können speziell für den einzelnen Anwendungsfall optimiert werden im Bezug auf
   - ihre Trenneigenschaften, d.h. Durchlässigkeit für die stoffwechselphysiologisch essentiellen Substanzen bei gleichzeitiger Undurchlässigkeit für Immunglobuline;
   - ihre Diffusionseigenschaften, d.h. rascher und ungehinderter Durchgang der essentiellen Substanzen;
   - die Durchmesser der Kapillarmembranen, die an die Größe der Zellen bzw. Zellinseln angepaßt sind, die in die Kapillare eingefüllt werden. Optimal ist der Kapillardurchmesser geringfügig größer als die Zell- bzw. Zellinselgröße.
2) Der Stoffaustausch des beschriebenen Systems beruht wie auch bei den bereits bekannten Systemen auf Diffusion. Die Diffusion ist abhängig von der Diffusionsstrecke und vom Konzentrationsgefälle. Das erfindungsgemäße Hohlorgan bietet minimale Diffusionsstrecken und infolge des unmittelbaren Kontakts von Membran und Transportmedium, z.B. Blut, einen optimalen Konzentrationsgradient.
3) Die auf die innenseitige, dem Blut zugewandte, Membranoberfläche, insbesondere Prothesenoberfläche vor der Implantation aufgesiedelte Zell-, insbesondere Endothelschicht stellt die natürliche, voll funktionsfähige Grenzschicht zum Blutstrom dar. Sie verhindert in optimaler Weise die sonst, insbesondere an der Grenzschicht Blut / künstliches Organ, auftretenden Komplikationen wie Thrombogenität, Komplementaktivierung u.a.
4) Der reversible Verschluß der Kammern, insbesondere der Kapillarzuführungen, und ihre Ausleitung in leicht zugängliche Hohlräume des Körpers oder durch die Haut des Patienten nach außen ermöglicht eine einfache Erneuerung der eingeführten Zellmasse bei auftretenden Komplikationen wie mangelnde Syntheseleistungen oder Zelltod, ohne daß ein komplizierter Eingriff und die Erneuerung des kompletten Implantats notwendig ist.
5) Einsatz von insbesondere bei Gefäßprothesen bekannten textilen Strukturen und Materialien als Träger.

Gemäß einer besonders bevorzugten Ausführungsform betrifft die Erfindung ein biohybrides Organ mit einem Träger, auf dem an der Innenseite, der Blutströmung zugewandt, mindestens ein hohles Membransystem angeordnet ist, in das funktionelle Zellen eingebracht sind, die in situ ihrerseits die physiologische, zellspezifische Wirkung zeigen, und auf dessen Membranoberfläche, zur Verhinderung der Ausbildung eines thrombotischen Belages, Endothelzellen aufgebracht sind, ohne daß diese die Diffusion durch die Membransysteme sonderlich beeinträchtigen.

### Anwendungsbeispiele

1) Hohlorgan in Form einer Gefäßprothese mit kreisförmigem Durchmesser von ca. 10 mm. Textiler Träger aus Faservlies gemäß Patent DE 2806030, hergestellt aus Polyurethan. Membransystem an der Innenseite des Hohlorgans, dem Blutstrom zugewandt, aus Polysulfon-Kapillaren mit kreisförmigem Durchmesser. Die Verbindung der Kapillarmembran mit dem textilen Träger erfolgt durch adhäsives Verkleben der Polyurethanfasern. Innendurchmesser der Kapillaren ca. 500 - 600 µm, Wandstärke ca. 100 - 150 µm. Wendelförmige Anordnung einer Kapillarmembran mit einem Zwischenraum von einer Kapillardicke zwischen den Windungen. Molekularer Cut-Off der Membran 80 kD. Beschichtung der innenseitigen, dem Blutstrom zugewandten, Prothesenoberfläche mit Fibronectin, anschließend Besiedlung mit körpereigenen Endothelzellen. In vitro-Kultivierung bis zur Erreichung eines stabilen, die gesamte Innenfläche der Prothese bedeckenden Zellrasens. Befüllung der Kapillarmembran mit Langerhans'schen Inseln. Verschluß der Kapillarzuführungen durch formschlüssiges, reversibles Abdichten mit einem Stopfen. Implantation der Prothese als A-V-Shunt, die Zuführungen der Kapillarmembran werden durch die Haut des Patienten nach außen geführt.
2) Hohlorgan in Form einer Gefäßprothese mit kreisförmigem Durchmesser von ca. 15 mm. Textiler Träger aus Gewebe, hergestellt aus thermoplastischem Polyester. Zwei unterschiedliche Kapillarmembransysteme an der Innenseite des Hohlorgans, dem Blutstrom zugewandt, bestehend aus Polycarbonat, mit kreisförmigem Durchmesser. Membransystem 1: Innendurchmesser der Kapillaren ca. 500 - 600 µm, Wandstärke ca. 100 - 150 µm. Membransystem 2: Innendurchmesser der Kapillaren 100 µm, Wandstärke ca. 100 - 150 µm. Ringförmige, vorzugsweise parallele wendelförmige Anordnung einer Vielzahl einzelner Windungen von Kapillarmembranen an der Innenwandung der Prothese, abwechselnd Membransystem 1 und Membransystem 2, ohne Zwischenraum zwischen den einzelnen Kapillarmembranen. Molekularer Cut-Off beider Membransysteme ca. 100 kD. Beschichtung der innenseitigen, dem Blutstrom zugewandten, Prothesenoberfläche mit Kollagen Typ IV, anschließend Besiedlung mit embryonalen Endothelzellen. In vitro-Kultivierung bis zur Erreichung eines stabilen, die gesamte Innenfläche der Prothese bedeckenden Zellrasens. Befüllung der Kapillarmembranen von Membransystem 1 mit Langerhans'schen Inseln, Membransystem 2 mit Hepatocyten. Verschluß der Kapillarzuführungen durch reversibles formschlüssiges Abdichten mit einem Stopfen. Implantation als Gefäßprothese, die Zuführungen der Kapillarmembranen werden durch die Haut des Patienten nach außen geführt.

Weitere Merkmale der Erfindung ergeben sich aus der nachfolgenden Beschreibung von bevorzugten Ausführungsformen in Verbindung mit der Zeichnung und den Unteransprüchen. Bei den einzelnen Ausführungsformen können die einzelnen Merkmale jeweils für sich alleine oder in Kombination miteinander verwirklicht sein.

In der Zeichnung zeigen:
- Figur 1:: einen Längsschnitt durch eine Ausführungsform, die jedoch nicht zur Erfindung gehört, sondern zum Verständis der weiteren Ausführungen dient.
- Figur 2:: einen Querschnitt durch die Ausführungsform nach Figur 1,
- Figur 3:: einen Längsschnitt durch eine Ausführungsform der Erfindung,
- Figur 4:: einen Längsschnitt durch eine weitere Ausführungsform der Erfindung,
- Figur 5:: einen Querschnitt durch die Ausführungsform nach Figur 4,
- Figur 6:: einen Längsschnitt durch eine weitere Ausführungsform der Erfindung.

Bei den einzelnen Figuren sind einander im wesentlichen entsprechende Bestandteile der Ausführungsformen mit den selben Bezugszeichen versehen.

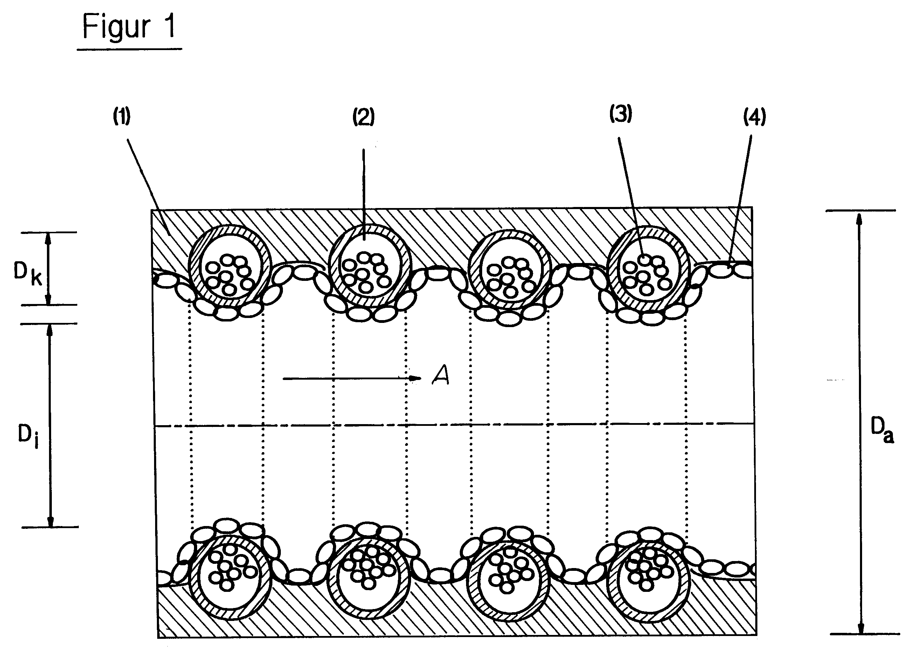

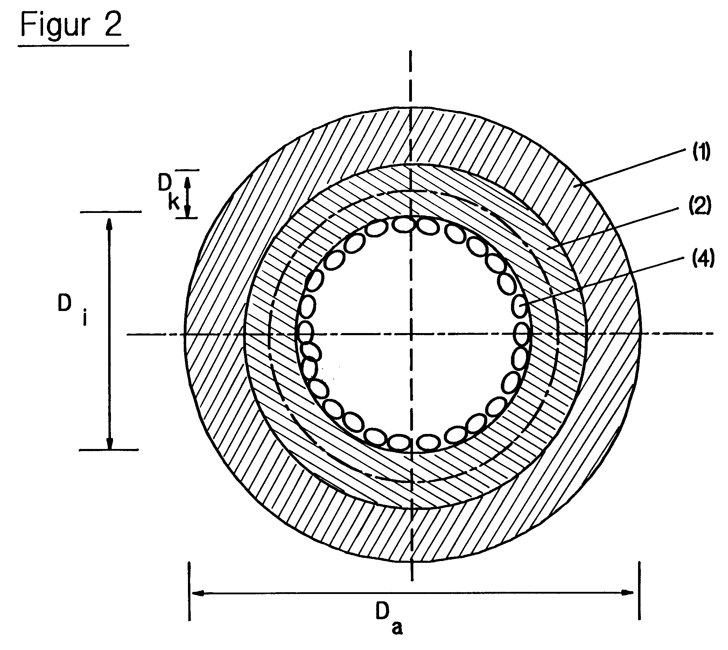

Die nicht zur Erfindung gehörende Ausführungsform nach den Figuren 1 und 2 zeigt ein biohybrides Organ in Form einer Gefäßprothese. Die Gefäßprothese setzt sich aus einem textilen Träger 1 zusammen, der den Grundkörper der Prothese bildet. Die Innenseite des Trägers 1 ist mit einer Vielzahl von ringförmigen Kapillarmembranen 2 bestückt, die am Innenumfang parallel zueinander verlaufen. Die ringförmigen Kapillarmembranen bilden sowohl die Kammern als auch die porösen Membranwandungen. Die ringförmigen Kapillarmembranen sind an ihrem Außenumfang mit der Innenseite des textilen Trägers durch Verklebung verbunden, so daß nur die dem Innenraum der Gefäßprothese zugewandte Oberfläche der Kapillarmembranen als poröse Membran zur Verfügung steht. Im Innern der Kapillarmembranen befinden sich Organzellen 3 in einem geeigneten flüssigen Medium. Die gesamte Innenseite der Gefäßprothese einschließlich der Kapillarmembranen ist mit einem Zellrasen aus Endothel-Zellen belegt, die einer Thrombogenität der Membranoberfläche entgegenwirken, gleichzeitig aber in einem so losen Verbund nebeneinader liegen, daß die Porosität der Kapillarmembranen nicht beeinträchtigt ist. Die Porengröße der Kapillarmembranen liegt im Bereich von 80 bis 100 kD, dies ermöglicht einen regen Austausch der durch die Organzellen gebildeten Wirkstoffe, ohne jedoch eine Immunreaktion zuzulassen, da die Porengröße das Eindringen von Immunglobulinen verhindert.

Der Außendurchmesser Dₐ der Gefäßprothese beträgt ca. 10 mm. Der Innendurchmesser Dᵢ liegt bei ca. 7 mm. Die Wandstärke Dₖ beträgt ca. 1,5 mm, wobei der Innendurchmesser der Kapillarmembranen ca. 1 mm beträgt.

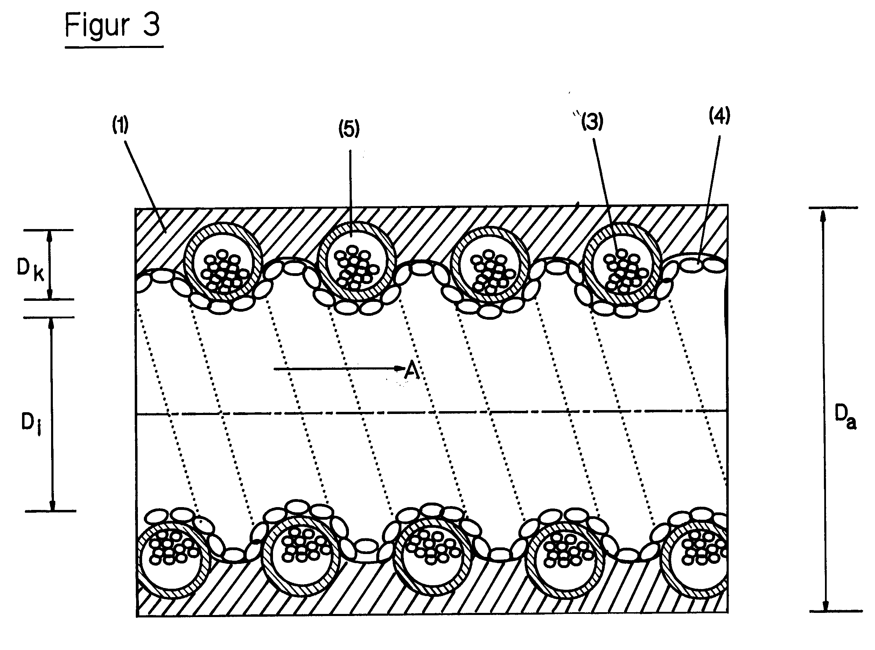

Die Ausführungsform nach Figur 3 entspricht im wesentlichen der Ausführungsform nach den Figuren 1 und 2. Im Unterschied dazu ist im Gegensatz zu der Anordnung einer Vielzahl von voneinader getrennten kapillarförmigen Ringen eine einzige langgestreckte rohrförmige Kapillarmembran vorgesehen, die wendelförmig bzw. nach Art einer Helix am Innenumfang des textilen Trägers verläuft. Es ist auch möglich zwei oder mehr Kapillarmembranen nach Art einer Doppelhelix parallel nebeneinander in ebenfalls einschichtiger Anordnung vorzusehen. Die Ausführungsform nach Figur 3 hat gegenüber der Ausführungsform nach den Figuren 1 und 2 den Vorteil, daß die einzelnen von den Windungen der Wendel gebildeten Kammerabschnitte miteinander in Verbindung stehen. Dadurch kann ein Austausch der Organzellen durch Spülung und Neubefüllung auch nach der Implantation in einfacher Weise vorgenommen werden. Hierzu dienen Anfang und Ende der wendelförmigen Kapillarmembran bzw. der Kapillarmembranen als Zuführung bzw. Ableitung und werden in entsprechender Verlängerung an von außen zugänglichen Körperstellen plaziert.

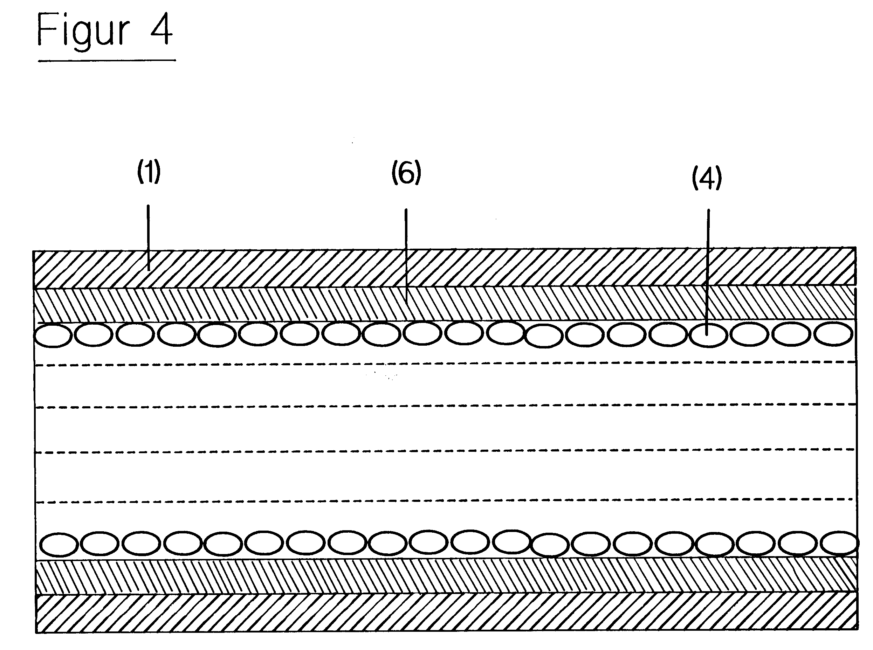

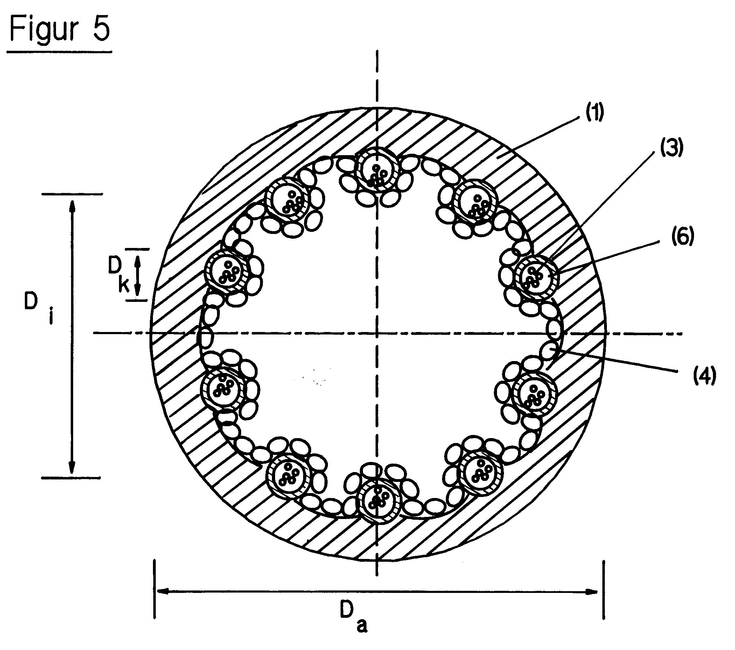

Die Ausführungsform nach den Figuren 4 und 5 zeigt eine axiale Anordnung von Kammern bzw. Kapillarmembranen am Innenumfang eines schlauchförmigen Trägers. Auch hier ist die gesamte Innenseite des Hohlorgans mit einer körpereigenen Zellschicht belegt, um Immunreaktionen zu vermeiden. Diese Ausführungsform eignet sich besonders dann, wenn die Kapilals getrennte Kammern dienen und verschiedene Organzellen enthalten. Auch hier ist eine Herausführung von Zu- und Ableitungen in Form von Verlängerungen der Kapillarmembranen möglich. Die Kapillarmembranen sind vorzugsweise als selbsttragende poröse und elastische Schläuche ausgebildet, wobei eine relativ grob poröse Wandung an ihrer Innenseite mit der eigentlichen feinporigen Membran versehen ist.

Wie aus der Zeichnung zu ersehen, sind die Kapillarmembranen bei den Ausführungsformen nach den Figuren 1 bis 5 in einem Abstand voneinander angeordnet, der im wesentlichen dem Außendurchmesser der Kapillarmembranen entspricht. Es ist aber auch möglich, die Kapillarmembranen so dicht nebeneinander anzuordnen, daß sie sich im wesentlichen gegenseitig berühren.

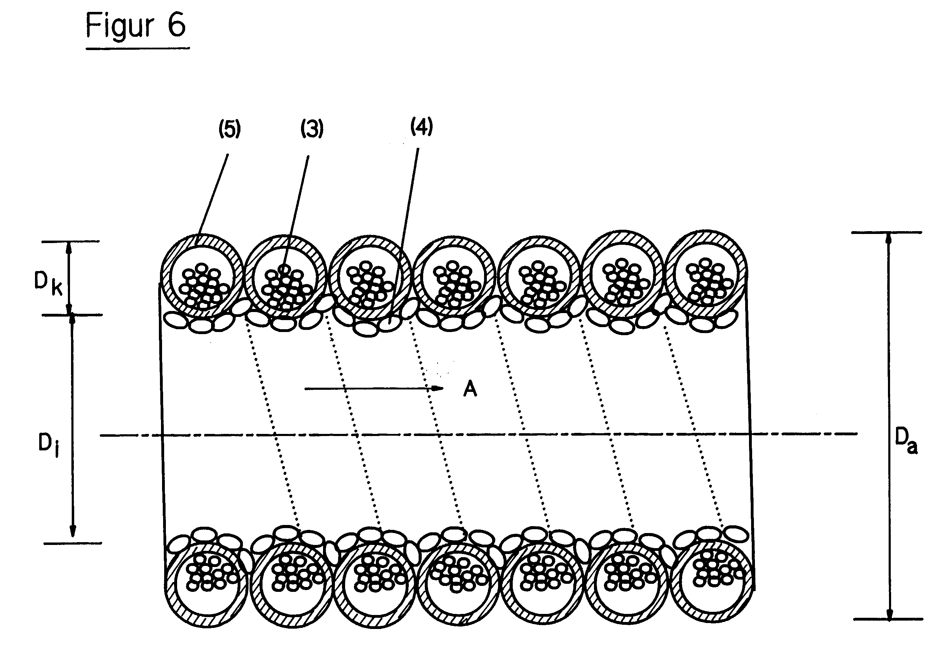

In Figur 6 ist eine derartige Ausführungsform dargestellt, bei der das Hohlorgan im wesentlichen ausschließlich von den einzelnen Kammern bzw. den hierzu vorgesehenen Kapillarmembranen gebildet wird. Eine oder mehrere Kapillarmembranen sind wendelförmig in einschichtiger Anordnung dicht an dicht gewickelt und miteinander dicht verklebt. Dadurch wird eine Gefäßprothese gebildet, die ausschließlich aus Kammern besteht. Die nach der Innenseite gerichtete Oberfläche der Kapillarmembranen ist wiederum mit einem Zellrasen belegt. An der Außenseite genügt ein Verschluß der Poren der Kapillarmembranen durch Überziehen mit einer Versiegelungsschicht, die gleichzeitig zur Verklebung verwendet wird.

Wie in den einzelnen Figuren ersichtlich, wird die Strömung einer Körperflüssigkeit durch die Gefäßprothesen in Richtung des Pfeiles A durch die erfindungsgemäßen biohydriden Organe nicht behindert. Trotzdem kann ein intensiver Stoffaustausch zwischen dem in den Kammern befindlichen Medium für die Organzellen und der durch das Hohlorgan strömenden Flüssigkeit stattfinden. Wenn es erwünscht ist, können zusätzlich Verjüngungen, insbesondere konische Verjüngungen im Hohlorgan vorgesehen sein, um durch Erzeugen einer Turbulenz eine weitere Erhöhung der Diffusionsgeschwindigkeit herbeizuführen.

Die Erfindung ist nicht auf die dargestellten Ausführungsformen beschränkt. Vielmehr sind Abweichungen davon möglich, ohne daß der Rahmen der Erfindung verlassen wird. So können die Hohlorgane auch so ausgebildet sein, daß sie nicht einen ganzen Abschnitt eines Gefäßes als Gefäßprothese ersetzen oder einen neuen Gefäßabschnitt, z.B. bei einem Bypass oder einem A-V-Shunt, bilden. Vielmehr ist es auch möglich, die Hohlorgane als Wandungsabschnitt auszubilden, der an der Innenseite eines bestehenden Gefäßes befestigt wird, oder einen entsprechend großen Wandabschnitt eines Gefäßes, der zuvor in Form eines Fensters entfernt wurde, zu ersetzen.

Weiterhin ist auch möglich, die Kammern an der Innenseite des Hohlorgans mit Flachmembranen auszubilden. Es kann auch ein schlangenlinienförmiger oder mäanderförmiger Verlauf von miteinander zusammenhängenden Kammerabschnitten vorgesehen sein. Bei besonderen Ausführungsformen ist es auch möglich, leitungsförmige Kammern über Kreuz zu verlegen, beispielsweise in Form einer gegenläufigen, sich überkreuzenden Doppelhelix oder in Form von insbesondere schlauchförmig ausgebildeten Geweben oder Geflechten von Kapillarmembranen, wobei die Kapillarmembranen auch in Form von parallelen Scharen verlegt sein können.

Die Herstellung der erfindungsgemäßen Hohlorgane kann in einfacher Weise vorgenommen werden, indem die Kammern, beispielsweise in Form von Kapillarmembranen auf einem Kern, beispielsweise einem Wachsdorn, verlegt werden. Die Kammern können dann beispielsweise durch gegenseitige Verklebung und Versiegelung an der Rückseite in ihrer Lage fixiert werden. Es ist auch möglich, auf die Außenseite der Kammern einen Träger, beispielsweise in Form eines textilen Faservlieses, aufzubringen. Nach Entfernen des Dornes bleibt das fertige Hohlorgan zurück.

### Literaturübersicht

1) Altman et al
   Implantation of semi-permeable hollow fibers to prevent immune rejection of transplanted pancreatic islets
   Horm. Met. Res. Suppl. 12 (1982), 43 - 45
2) Araki Y. et al
   Normalization of blood glucose in totally pancreatectomized dogs by use of pancreatic chambers
   Endocrinol. Japan. 27, 2 (1980), 157 - 161
3) Bretzel, R.G.
   Workshop on "Immunoisolation of Islets of Langerhans", 1988
4) Chick W.L. et al
   Beta cell culture on synthetic capillaries: An artificial endocrine pancreas
   Science 187 (1975), 847 - 849
5) Darquy S. et al
   Comparative study of microencapsulated rat islets implanted in different diabetic models in mice
   Methods in Islet Transplantation Research, Thieme (1990), 209 - 214
6) Dupuy B. et al
   In situ polymerisation of a microencapsulating medium round living cells
   J. Biomed. Mater. Res. 22 (1988), 1061 - 1070
7) Iwata H. et al
   Evaluation of encapsulated Langerhans islets in polyion complex microbeads as a bioartificial pancreas
   Transactions 3rd World Biomaterials Congress Kyoto (1988), 291
8) Klomp, G.F. et al
   Hydrogels for encapsulation of pancreatic islet cells
   Trans. Am. Soc. Artif. Inter. Organs 25 (1979), 74 - 76
9) Lim F. et al
   Microencapsulated islets as bioartificial endocrine pancreas
   Science 210 (1980), 908 - 912
10) Matsumura K.N. et al
   Hybrid artificial liver in hepatic failure. Preliminary clinical report
   Surgery 101, 1 (1987), 99 - 103
11) Naber S.P. et al
   Progress in development of a hybrid artificial endocrine pancreas
   Toxl. Med. Int. Congr. Series 580 (1980), 227 - 231
12) Reach G. et al
   Functional evaluation of a bioartificial pancreas using isolated islets perifused with blood ultrafiltrate
   Diabetes 30 (1981), 296 - 301
13) Reach G. et al
   A u-shaped bioartificial pancreas with rapid glucose-insulin kinetics. In vitro evaluation and kinetic modelling
   Diabetes 33 (1984), 752 - 761
14) Sefton M.V. et al
   Submerged extrusion for the microencapsulation of mammalian cells in poly-HEMA-MMA
   Transactions 3rd World Biomaterials Congress Kyoto (1988), 294
15) Strautz R.L.
   Studies of hereditary-obese mice after implantation of pancreatic islets in millipore filter capsules
   Diabetol. 6 (1970), 306 - 312
16) Sun A.M. et al
   The use, in diabetic rats and monkeys, of artificial capillary units containing cultured islets of Langerhans (artificial endocrine pancreas)
   Diabetes 26 (1977), 1136 - 1139
17) Sun A.M. et al
   Microencapsulated hepatocytes as a bioartificial liver
   Trans. Am. Soc. Artif. Int. Organs 32 (1986), 39 - 41
18) Tze W.J. et al
   Implantable artificial capillary unit for pancreatic islet allograft and xenograft
   Diabetol. 16 (1979), 247 - 252
19) Tze W.J. et al
   Studies with implantable artificial capillary units containing rat islets on diabetic dogs
   Diabetol. 19 (1980), 541 -545
20) Valente U. et al
   Human pancreatic islet transplantation in diffusion chambers
   Horm. Met. Res. Suppl. 12 (1982), 48 - 51
21) Wolf C.F.W.
   Cells cultured on artificial capillaries and their use as a liver assist device
   Artif. Organs 4,4 (1980), 279 - 284
22) Zekorn T. et al
   Islet transplantation in experimental diabetes of the rat. XI. in vitro tests on artificial membranes applied in islet transplantation
   Horm. Met. Res. 19 (1987), 87 - 88
23) Zekorn T. et al
   Bioartificial pancreas: The use of different hollow fibers as a diffusion chamber
   Transplantation Proceedings 21 (1989), 2748 - 2750

## Patentansprüche

1. Biohybrides Hohlorgan mit mindestens einer rohrförmigen Kammer (2; 5) mit einem kreisförmigen, ovalen, trilobalen, triangel- oder trapezförmigen Querschnitt von 0,001-15 mm² zur Aufnahme von Organzellen (3), wobei die Kammerwandungen mindestens teilweise von einer mikro-porösen Membran gebildet werden, wobei das Hohlorgan zum Einsatz als Wandungsabschnitt einer Leitungsbahn beim Empfänger ausgebildet ist und die Membran sich mindestens an der dem Innenraum der Leitungsbahn zugewandten Innenseite der Wandung des Hohlorgans befindet, dadurch gekennzeichnet, daß Anfang und Ende der mindestens einen Kammer derart als Zuführung bzw. Ableitung ausgebildet sind, daß die mindestens eine Kammer durchspülbar ist.

2. Hohlorgan nach Anspruch 1, dadurch gekennzeichnet, daß mindestens eine Kammer (2; 5) auf einem Träger (1) angeordnet ist, der vorzugsweise den Wandabschnitt, insbesondere Längsabschnitt, der Leitungsbahn bildet, und vorzugsweise als Prothese ausgebildet ist.

3. Hohlorgan nach Anspruch 1 oder 2, dadurch gekennzeichnet, daß der lichte Abstand zwischen Kammerwandungen, zumindest in Richtung auf das Innere der Leitungsbahn gesehen, mindestens das 1,1-fache, vorzugsweise das 1,5-bis 20-fache, insbesondere das 2- bis 5-fache der Größe der aufzunehmenden Organzellen bzw. Organzellinseln beträgt.

4. Hohlorgan nach einem der vorhergehenden Ansprüche, dadurch gekennzeichnet, daß mindestens eine Kammer (2; 5) nach Art einer Leitung ausgebildet ist.

5. Hohlorgan nach einem der vorhergehenden Ansprüche, dadurch gekennzeichnet, daß mindestens eine Kammer (2; 5) als eine Leitung ausgebildet ist, deren Länge im Verhältnis zum Innendurchmesser der Leitung groß ist, vorzugweise größer 10 : 1, insbesondere größer 100 : 1.

6. Hohlorgan nach einem der vorhergehenden Ansprüche, dadurch gekennzeichnet, daß die Membran eine Wandstärke zwischen 1 µm und 300 µm, insbesondere zwischen 10 µm und 200 µm besitzt.

7. Hohlorgan nach einem der vorhergehenden Ansprüche, insbesondere nach Anspruch 2, dadurch gekennzeichnet, daß Kammerabschnitte bzw. Kammern von einzelnen Leitungs-, insbesondere Kapillarmembranen gebildet werden, die an der Innenseite eines Trägers (1) angeordnet sind.

8. Hohlorgan nach einem der vorhergehenden Ansprüche, dadurch gekennzeichnet, daß mehrere Kammerabschnitte bzw. Kammern vorgesehen sind, die einen Abstand größer 0,1 mm, insbesondere einen Abstand in der Größenordnung des Durchmessers einer Leitungsmembran voneinander haben.

9. Hohlorgan nach einem der vorhergehenden Ansprüche, dadurch gekennzeichnet, daß mindestens eine Kammer, vorzugsweise sämtliche Kammern an ihren Enden verschließbare Öffnungen besitzen.

10. Hohlorgan nach einem der vorhergehenden Ansprüche, dadurch gekennzeichnet, daß es zum Einsatz an eine stark durchblutete Stelle des Gefäßsystemes ausgebildet ist, insbesondere als A-V-Shunt.

11. Hohlorgan nach einem der vorhergehenden Ansprüche, dadurch gekennzeichnet, daß es mindestens im Bereich der Kammermembran eine Einrichtung zur Erzeugung einer Turbulenz einer durch die Leitungsbahn fließenden Flüssigkeit aufweist, insbesondere eine eine konische Verengung der Leitungsbahn bildende Struktur aufweist.

## Claims

1. Biohybrid hollow organ with at least one tubular chamber (2, 5) with a circular, oval, trilobal, triangular or trapezoidal cross-section of 0.001 to 15 mm² for receiving organ cells (3), the chamber walls being at least partly formed by a microporous membrane, in which the hollow organ is constructed for use as a wall portion of a conducting path in the donee and the membrane is at least located on the inside of the wall of the hollow organ facing the interior of the conducting path, characterized in that the beginning and end of at least one chamber are constructed as a feed or drain line in such a way that the at least one chamber can be irrigated.

2. Hollow organ according to claim 1, characterized in that at least one chamber (2, 5) is placed on a support (1), which preferably forms the wall portion, particularly the longitudinal portion of the conducting path and is preferably constructed as a prosthesis.

3. Hollow organ according to claim 1 or 2, characterized in that the internal spacing between the chamber walls, at least in the direction towards the interior of the conducting path is at least 1.1, preferably 1.5 to 20 and in particular 2 to 5 times the size of the organ cells or organ cell islets to be received.

4. Hollow organ according to one of the preceding claims, characterized in that at least one chamber (2, 5) is constructed in the manner of a line.

5. Hollow organ according to one of the preceding claims, characterized in that at least one chamber (2, 5) is constructed as a line, whose length is large compared with the internal diameter of the line, preferably larger than 10:1 and in particular larger than 100:1.

6. Hollow organ according to one of the preceding claims, characterized in that the membrane has a wall thickness between 1 and 300, particularly between 10 and 200 µm.

7. Hollow organ according to one of the preceding claims, particularly according to claim 2, characterized in that chamber portions or chambers are formed by individual line and in particular capillary membranes located on the inside of a support (1).

8. Hollow organ according to one of the preceding claims, characterized in that there are several chamber portions or chambers, which have a reciprocal spacing greater than 0.1 mm and in particular a spacing of the same order of magnitude as a line membrane diameter.

9. Hollow organ according to one of the preceding claims, characterized in that at least one chamber and preferably all the chambers have closable openings on their ends.

10. Hollow organ according to one of the preceding claims, characterized in that it is constructed for use at a point of the vascular system through which there is a strong blood circulation, more particularly as an A-V-shunt.

11. Hollow organ according to one of the preceding claims, characterized in that at least in the vicinity of the chamber membrane, there is a device for producing turbulence in a liquid flowing through the conducting path, particularly a structure forming a conical constriction of said path.

## Revendications

1. Organe creux biohybride comportant au moins une chambre tubulaire (2;5) présentant une section circulaire, ovale, trilobée, triangulaire ou trapézoïdale, de 0,001-15 mm², pour recevoir des cellules d'organe (3), les parois des chambres ou cavités étant formées au moins en partie par une membrane microporeuse, l'organe creux étant conformé pour servir de tronçon de paroi d'un organe de conduction chez le receveur et la membrane se trouvant au moins sur le côté intérieur de la paroi de l'organe creux tourné vers l'espace intérieur de l'organe conducteur, organe caractérisé en ce que le début et la fin de ladite au moins une chambre ou cavité sont configurés en des conduits d'acheminement ou d'évacuation de sorte qu'au moins une chambre ou cavité peut être irriguée.

2. Organe creux selon la revendication, caractérisé en ce qu'au moins une chambre ou cavité (2;5) est disposée sur un support (1), qui forme avantageusement le tronçon de paroi, notamment un tronçon longitudinal, de l'organe conducteur et est avantageusement conformé en prothèse.

3. Organe creux selon la revendication 1 ou 2, caractérisé en ce que la distance libre ou lumière entre les parois de chambre, vue au moins dans le sens de l'intérieur du trajet ou organe conducteur, représente au moins 1,1 fois, avantageusement 1,5 à 20 fois, notamment 2 à 5 fois, la grandeur des cellules d'organes ou des îlots de cellules d'organe à y recevoir.

4. Organe creux selon l'une des revendications précédentes, caractérisé en ce qu'au moins une chambre (2;5) est conformée en un genre de conduit.

5. Organe creux selon l'une des revendications précédentes, caractérisé en ce qu'au moins une chambre ou cavité (2;5) est conformé en un conduit dont la longueur est grande par rapport au diamètre intérieur de ce conduit, qui présente avantageusement un rapport supérieur à 10:1 notamment supérieur à 100:1.

6. Organe creux selon l'une des revendications précédentes, caractérisé en ce que la membrane possède une épaisseur de paroi comprise entre 1 µm et 300 µm, notamment entre 10 µm et 200µm.

7. Organe creux selon l'une des revendications précédentes, notamment selon la revendication 2, caractérisé en ce que les tronçons de chambre ou chambres sont formés de membranes conductrices individuelles, en particulier de membranes capillaires, qui sont disposées sur le côté intérieur d'un support (1).

8. Organe creux selon l'une des revendications précédentes, caractérisé en ce que l'on prévoit plusieurs tronçons de chambre ou plusieurs chambres présentant une distance mutuelle supérieure à 0,1 mm, notamment une distance ayant l'ordre de grandeur du diamètre d'une membrane conductrice.

9. Organe creux selon l'une des revendications précédentes, caractérisé en ce qu'au moins une chambre et avantageusement toutes les chambres possède(nt) à ses ou a leurs extrémités des ouvertures ou orifices obturables.

10. Organe creux selon l'une des revendications précédentes, caractérisé en ce qu'il est conformé pour être placé en un endroit fortement vascularisé du système des vaisseaux, notamment comme pontage ou "shunt" A-V.

11. Organe creux selon l'une des revendications précédentes, caractérisé en ce qu'il présente, au moins dans la zone de membrane de chambre, un dispositif pour produire une turbulence d'un liquide circulant dans le trajet conducteur, notamment une structure formant un rétrécissement conique du trajet conducteur.
